(19) Europäisches Patentamt / European Patent Office / Office européen des brevets

(11) **EP 4 501 308 A1**

(12) **EUROPEAN PATENT APPLICATION**
published in accordance with Art. 153(4) EPC

(43) Date of publication:
**05.02.2025 Bulletin 2025/06**

(21) Application number: 23774288.7

(22) Date of filing: **10.02.2023**

(51) International Patent Classification (IPC):
*A61K 8/46* (2006.01)   *A61K 8/34* (2006.01)
*A61K 8/60* (2006.01)   *A61K 8/86* (2006.01)
*A61Q 5/02* (2006.01)

(52) Cooperative Patent Classification (CPC):
A61K 8/34; A61K 8/46; A61K 8/60; A61K 8/86;
A61Q 5/02

(86) International application number:
**PCT/JP2023/004498**

(87) International publication number:
**WO 2023/181689 (28.09.2023 Gazette 2023/39)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **24.03.2022  JP 2022048618**

(71) Applicant: **Kao Corporation
Tokyo 103-8210 (JP)**

(72) Inventor: **TORII, Shusaku
Tokyo 131-8501 (JP)**

(74) Representative: **Hoffmann Eitle
Patent- und Rechtsanwälte PartmbB
Arabellastraße 30
81925 München (DE)**

(54) **HAIR WASHING COMPOSITION**

(57)   The present invention relates to a hair cleansing composition containing a specific internal olefin sulfonic acid or a salt thereof and, when applied to hair, exhibits excellent performance from the time of cleansing to the time of rinsing and after cleansing. That is, the present invention is a hair cleansing composition containing the following components (A) and (B): (A) an internal olefin sulfonic acid having 16 carbon atoms or a salt thereof obtained by sulfonation of a raw material olefin having 16 carbon atoms and having an average double bond position of 3.9-position or more and 4.4-position or less; and (B) one or more hydroxy group-containing compounds selected from the group consisting of (b1) a monosaccharide or a sugar alcohol and (b2) polyethylene glycol having a mass average molecular weight of 500 or less, wherein a mass ratio of a content of the component (B) to a content of the component (A), (B)/(A), is 0.005 or more and 0.45 or less.

**EP 4 501 308 A1**

**Description**

Field of the Invention

The present invention relates to a hair cleansing composition.

Background of the Invention

Internal olefin sulfonates, which are known to have various numbers of carbon atoms, can achieve excellent effects as anionic surfactants and therefore have been conventionally used in various cleansing compositions.

For example, Patent Literature 1 discloses a liquid detergent composition for textile products, the composition containing a sulfonate of an internal olefin having from 12 to 24 carbon atoms, a fatty acid or a salt thereof, an organic solvent having a hydroxy group, and water in a specific quantitative relationship, and the composition melts even after undergoing a large number of freeze-thaw cycles in which the composition once being frozen is caused to melt, and thus maintains a good appearance.

Patent Literature 2 discloses a liquid detergent composition for textile products, the composition containing an internal olefin sulfonate having from 14 to 16 carbon atoms, an organic solvent having a hydroxy group, and water in specific amounts, wherein the internal olefin sulfonate having a sulfonate group at 2-position or more and 4-position or less and the internal olefin sulfonate salt having a sulfonate group at 5-position or more are in a specific mass ratio, and production and separation of solids are suppressed even when the composition is exposed to a low-temperature environment, giving improved detergency.

Citation List

Patent Literatures

Patent Literature 1: JP-A-2020-109145
Patent Literature 2: JP-A-2017-214571

Summary of the Invention

The present invention provides a hair cleansing composition containing the following components (A) and (B):

(A) an internal olefin sulfonic acid having 16 carbon atoms or a salt thereof obtained by sulfonation of a raw material olefin having 16 carbon atoms and having an average double bond position of 3.9-position or more and 4.4-position or less; and
(B) one or more hydroxy group-containing compounds selected from the group consisting of (b1) a monosaccharide or a sugar alcohol and (b2) polyethylene glycol having a mass average molecular weight of 500 or less,

wherein a mass ratio of a content of the component (B) to a content of the component (A), (B)/(A), is 0.005 or more and 0.45 or less.

The internal olefin sulfonate is a component capable of imparting such various excellent performance to the composition. However, for hair cleansing, none of the above Patent Literatures are concerned with imparting a delicate finish to hair not only at the time of cleansing but also at the time of rinsing and after cleansing, and, accordingly, there is sufficient room for improvement.

The present invention relates to a hair cleansing composition which contains a specific internal olefin sulfonic acid or a salt thereof and, when applied to hair, exhibits excellent performance from the time of cleansing to the time of rinsing and after cleansing.

The present inventor conducted various studies and found that a hair cleansing composition exhibiting excellent hair cleansing effect from the time of cleansing to the time of rinsing and after cleansing can be obtained by containing a specific internal olefin sulfonic acid or a salt thereof, obtained by sulfonation of a raw material olefin having an average double bond position within a limited range and a specific hydroxy group-containing compound in a specific mass ratio.

The hair cleansing composition of the present invention when applied to hair provides good foaming at the time of cleansing, and not only enables the softness of hair to be felt at the time of rinsing but also enables, because the hair cleansing composition unlikely causes wet hair after cleansing to entangle or unlikely creates an undesired bundle feel, good combability to be felt. Also, the composition has good low-temperature stability and is thus highly useful.

The "good foaming" at the time of cleansing hair provided by the hair cleansing composition of the present invention means that the volume of foam is sufficient for experiencing the expression of foaming power which causes foam to be promptly formed and for experiencing a dirt removing effect when cleansing hair. The "softness of hair" at the time of rinsing after cleansing hair with the hair cleansing composition of the present invention means that not only the softness and suppleness of the whole hair can be felt at the time of rinsing but also the flexibility and healthiness of each strand of hair at the time of finishing can be felt. In addition, the "good combability" of wet hair after cleansing means that hair is prevented from becoming unmanageable by being entangled or creating an undesired bundle feel, aligned hair flow can be spontaneously provided without applying an extra load by hand combing or the like, and thus brushing and subsequent drying are easily performed. Here the "bundle feel" means an unpleasant feel resulting from that the cleansing composition remains on the hair surface, thus holds water between threads of hair, and causes each thread of hair to bundle together without being independent.

Hereinafter, these effects of "good foaming", "imparting softness to hair", and "imparting good combability" to wet hair after cleansing when the hair cleansing composition of the present invention is applied to hair are also collectively referred to as a "hair cleansing effect".

Detailed Description of the Invention

The present invention will now be described in detail.

The hair cleansing composition of the present invention contains, as a component (A), an internal olefin sulfonic acid having 16 carbon atoms or a salt thereof obtained by sulfonation of a raw material olefin having 16 carbon atoms and having an average double bond position of 3.9-position or more and 4.4-position or less. That is, the internal olefin sulfonic acid having 16 carbon atoms or a salt thereof as the component (A) is a compound that is obtained by using a raw material olefin having an average double bond position within a specific limited range as a starting raw material and sulfonating it, specifically, a compound that is obtained by sulfonating a raw material olefin and then neutralizing and hydrolyzing it.

Examples of the salt of the internal olefin sulfonic acid having 16 carbon atoms obtained by sulfonation of a raw material olefin having 16 carbon atoms and having an average double bond position of 3.9-position or more and 4.4-position or less include one or more selected from the group consisting of alkali metal salts, such as a sodium salt and a potassium salt; organic amine salts, such as an ammonium salt, a monoethanolamine salt, a diethanolamine salt, a triethanolamine salt, a 2-aminoethanol salt, and a 2-aminomethyl propanediol salt; and basic amino acid salts, such as a lysine salt and an arginine salt. These internal olefin sulfonates having 16 carbon atoms may not be necessarily in a salt form from the beginning and may be a salt that is generated by a neutralization reaction during manufacturing.

In particular, the salt of the internal olefin sulfonic acid having 16 carbon atoms is, from the viewpoint of effectively enhancing the hair cleansing effect together with the component (B) which will be described below and from the viewpoint of low-temperature stability, preferably one or more selected from the group consisting of a sodium salt, a potassium salt, an ammonium salt, and a 2-aminoethanol salt, more preferably one or two selected from the group consisting of a sodium salt and a potassium salt, and further more preferably a sodium salt. That is, sodium internal olefin sulfonate having 16 carbon atoms is further more preferred.

The internal olefin sulfonic acid having 16 carbon atoms or a salt thereof as the component (A), which is a product obtained from such a raw material olefin having 16 carbon atoms, is mainly a mixture of hydroxyalkanesulfonic acid having 16 carbon atoms or a salt thereof (hydroxy form, abbreviation: "HAS") and olefin sulfonic acid having 16 carbon atoms or a salt thereof (olefin form, abbreviation: "IOS").

In the raw material olefin having 16 carbon atoms as a starting raw material of the component (A), the positions of double bonds are mainly located inside the carbon chain, but a so-called α-olefin in which the double bond is located at the 1-position of the carbon chain may be contained in a trace amount. When such a raw material olefin is sulfonated, β-sultone is mainly produced, and a part of the β-sultone is converted into γ-sultone and an olefin sulfonic acid. These β-sultone, γ-sultone, and olefin sulfonic acid are further converted into hydroxyalkanesulfonic acid having 16 carbon atoms or a salt thereof and olefin sulfonic acid having 16 carbon atoms or a salt thereof in the neutralization/hydrolysis process (for example, J. Am. Oil Chem. Soc. 69, 39 (1992)). The hydroxy group of the resulting hydroxyalkanesulfonic acid or a salt thereof is located inside the alkane chain, and the double bond of the olefin sulfonic acid or a salt thereof is located inside the olefin chain.

Accordingly, in the present specification, these products and a mixture thereof are collectively referred to as an internal olefin sulfonic acid having 16 carbon atoms or a salt thereof as the component (A).

The raw material olefin having 16 carbon atoms that is sulfonated and forms an internal olefin sulfonic acid having 16 carbon atoms or a salt thereof as the component (A) has an average double bond position of 3.9-position or more and 4.4-position or less. The raw material olefin having 16 carbon atoms and having an average double bond position of such a value has a broad distribution of double bond positions over from the 2-position to the 8-position including the 1-position that can be contained in a trace amount.

The double bond positions and its distribution in a raw material olefin can be verified by measurement using a gas chromatography mass spectrometer (abbreviation: "GC-MS"). Specifically, each of components having different carbon chain lengths and double bond positions is precisely separated by a gas chromatography apparatus (hereinafter, abbreviated as GC), and the double bond positions can be identified by application of them to a mass spectrometer (abbreviation: "MS"), and the distribution is determined from the respective GC peak areas.

On the other hand, in the internal olefin sulfonic acid having 16 carbon atoms or a salt thereof as the component (A) obtained by sulfonation of such a raw material olefin, the more the position of the sulfonate group introduced by sulfonation is inside the carbon chain, the more difficult the separation. Accordingly, there is no definitive analytical method at present. However, the positions of the sulfonate groups in the component (A) are fully presumed to approximately correspond to the double bond positions in the raw material olefin and to show a broad distribution over from the 2-position to the 8-position including the 1-position without being excessively unevenly distributed. Accordingly, in the present invention, the component (A) is regulated based on the value of the average double bond position in the raw material olefin as a starting raw material.

The component (A) used in the present invention is an internal olefin sulfonic acid or a salt thereof obtained from a raw material olefin having the above-mentioned average double bond position value, i.e., a broad double bond distribution, and the sulfonate groups in the carbon chain are present in broad positions without being excessively unevenly distributed. In the internal olefin sulfonic acid having 16 carbon atoms or a salt thereof, when sulfonic acid groups are unevenly distributed more toward the carbon chain terminals, the melting point is increased due to the increased elongated carbon chains, thereby likely resulting in precipitation and also possibly deteriorating low-temperature stability. In contrast, if sulfonate groups are unevenly distributed near the interior of the carbon chain, the foam quality at the time of cleansing and the feel of hair at the time of rinsing are deteriorated, and the hair cleansing effect may be reduced. However, in the internal olefin sulfonic acid having 16 carbon atoms or a salt thereof as the component (A) obtained from the above-mentioned raw material olefin, sulfonate groups are present in broad positions without being excessively unevenly distributed in the carbon chain, the internal olefin sulfonic acids or salts thereof having various lengths of the carbon chain from the bonding position of the sulfonate group to the end are moderately mixed. Accordingly, the component (A), in cooperation with the component (B) which will be described below, can exhibit the excellent hair cleansing effect from the time of cleansing to the time of rinsing and after cleansing. The same applies also when the component (A) is sodium internal olefin sulfonate having 16 carbon atoms.

The average double bond position (unit: position, abbreviated as "DBP") in the raw material olefin having 16 carbon atoms as the starting raw material of the component (A) means the average value of the double bond positions of each raw material olefin having 16 carbon atoms present in the total amount of the raw material olefin having 16 carbon atoms. Specifically, the average double bond position of a raw material olefin having 16 carbon atoms is a value determined by the following expression (1).

$$\text{Average double bond position of raw material olefin having 16 carbon atoms} = \sum_{n=1}^{8} n \times C_n / 100 \qquad \cdots (1)$$

(In the expression (1), n represents an integer (unit: position) indicating the position of a double bond existing in a raw material olefin having 16 carbon atoms; and Cn represents the content (unit: mass%) of the raw material olefin having 16 carbon atoms and having a double bond at n-position in the total amount of 100 mass% of the raw material olefin having 16 carbon atoms.)

The average double bond position in the raw material olefin having 16 carbon atoms forming the component (A) is, from the viewpoint of securing the exhibition of excellent hair cleansing effect, 3.9-position or more and preferably 4.0-position or more, and is 4.4-position or less, preferably 4.3-position or less, and more preferably 4.2-position or less. In addition, the average double bond position in the raw material olefin having 16 carbon atoms is 3.9-position or more and 4.4-position or less, preferably from 4.0-position to 4.4-position, more preferably from 4.0-position to 4.3-position, and further more preferably from 4.0-position to 4.2-position.

In the raw material olefin having 16 carbon atoms, the content of a raw material olefin having a double bond position at the 2-position in the raw material olefin having 16 carbon atoms is preferably 10 mass% or more, more preferably 15 mass% or more, and further more preferably 20 mass% or more and preferably 35 mass% or less, more preferably 32 mass% or less, and further more preferably 24 mass% or less. In addition, in the raw material olefin having 16 carbon atoms, the content of the raw material olefin having a double bond position at the 2-position in the raw material olefin having 16 carbon atoms is preferably from 10 to 35 mass%, more preferably from 15 to 32 mass%, and further more preferably from 20 to 24 mass%.

In the raw material olefin having 16 carbon atoms, the content of a raw material olefin having a double bond position at the 3-position in the raw material olefin having 16 carbon atoms is preferably 10 mass% or more, more preferably 14 mass% or more, and further more preferably 16 mass% or more and preferably 30 mass% or less, more preferably 24 mass% or less, and further more preferably 19 mass% or less. In addition, in the raw material olefin having 16 carbon atoms, the content of the raw material olefin having a double bond position at the 3-position in the raw material olefin having 16 carbon atoms is preferably from 10 to 30 mass%, more preferably from 14 to 24 mass%, and further more preferably from 16 to 19 mass%.

In the raw material olefin having 16 carbon atoms, the content of a raw material olefin having a double bond position at the 4-position in the raw material olefin having 16 carbon atoms is preferably 10 mass% or more, more preferably 15 mass% or more, and further more preferably 17 mass% or more and preferably 30 mass% or less, more preferably 25 mass% or less, and further more preferably 19 mass% or less. In addition, in the raw material olefin having 16 carbon atoms, the content of the raw material olefin having a double bond position at the 4-position in the raw material olefin having 16 carbon atoms is preferably from 10 to 30 mass%, more preferably from 15 to 25 mass%, and further more preferably from 17 to 19 mass%.

In the raw material olefin having 16 carbon atoms, the content of a raw material olefin having a double bond position at the 5-position in the raw material olefin having 16 carbon atoms is preferably 5 mass% or more, more preferably 10 mass% or more, and further more preferably 13 mass% or more and preferably 25 mass% or less, more preferably 19 mass% or less, and further more preferably 15 mass% or less. In addition, in the raw material olefin having 16 carbon atoms, the content of the raw material olefin having a double bond position at the 5-position is preferably from 5 to 25 mass%, more preferably from 10 to 19 mass%, and further more preferably from 13 to 15 mass% in the raw material olefin having 16 carbon atoms.

In the raw material olefin having 16 carbon atoms, the content of a raw material olefin having a double bond position at the 6-position in the raw material olefin having 16 carbon atoms is preferably 5 mass% or more, more preferably 7 mass% or more, and further more preferably 11 mass% or more and preferably 20 mass% or less, more preferably 15 mass% or less, and further more preferably 13 mass% or less. In addition, in the raw material olefin having 16 carbon atoms, the content of the raw material olefin having a double bond position at the 6-position in the raw material olefin having 16 carbon atoms is preferably from 5 to 20 mass%, more preferably from 7 to 15 mass%, and further more preferably from 11 to 13 mass%.

In the raw material olefin having 16 carbon atoms, the total content of raw material olefins having a double bond position at the 7-position or 8-position in the raw material olefin having 16 carbon atoms is preferably 5 mass% or more, more preferably 7 mass% or more, and further more preferably 12 mass% or more and preferably 25 mass% or less, more preferably 22 mass% or less, and further more preferably 16 mass% or less. In addition, in the raw material olefin having 16 carbon atoms, the total content of the raw material olefins having a double bond position at the 7-position or 8-position in the raw material olefin having 16 carbon atoms is preferably from 5 to 25 mass%, more preferably from 7 to 22 mass%, and further more preferably from 12 to 16 mass%.

In the raw material olefin having 16 carbon atoms, the mass ratio of the content of raw material olefins having a double bond position at the 3- to 5-positions to the content of raw material olefins having a double bond position at the 6- to 8-positions, (raw material olefin $_{3\text{ to }5\text{-}}$ position/raw material olefin $_{6\text{ to }8\text{-}}$position), is preferably 1.0 or more, more preferably 1.3 or more, and further more preferably 1.7 or more and preferably 4.0 or less, more preferably 3.5 or less, and further more preferably 2.2 or less. In addition, in the raw material olefin having 16 carbon atoms, the mass ratio of the content of raw material olefins having a double bond position at the 3- to 5-positions to the content of raw material olefins having a double bond position at the 6- to 8-positions, (raw material olefin $_{3\text{ to }5}$-position/raw material olefin $_{6\text{ to }8\text{-}}$ position), is preferably from 1.0 to 4.0, more preferably from 1.3 to 3.5, and further more preferably from 1.7 to 2.2.

In the raw material olefin having 16 carbon atoms, the content of a raw material olefin having a double bond position at the 1-position ($\alpha$-olefin), which may inevitably exist, is preferably less than 5.0 mass%, more preferably less than 3.0 mass%, and further more preferably less than 2.5 mass% in the raw material olefin having 16 carbon atoms. Alternatively, the raw material olefin having 16 carbon atoms preferably does not contain $\alpha$-olefin.

The above-mentioned raw material olefin having 16 carbon atoms can be obtained by isomerization (double bond transfer) of a raw material olefin having a double bond position at the 1-position ($\alpha$-olefin) that is generated by dehydration reaction of an alcohol having 16 carbon atoms. Specifically, a solid acid catalyst, such as alumina, in an amount of preferably 0.5 parts by mass or more, more preferably 2 parts by mass or more, and preferably 15 parts by mass or less, more preferably 10 parts by mass or less, and preferably from 0.5 to 15 parts by mass, more preferably from 2 to 10 parts by mass, is added to 100 parts by mass of 1-hexadecanol.

Subsequently, isomerization reaction is performed by stirring at preferably 220°C or more, more preferably 260°C or more, and preferably 350°C or less, and preferably from 220°C to 350°C, more preferably from 260°C to 350°C, for preferably 1 hour or more, more preferably 3 hours or more, and preferably 30 hours or less, more preferably 10 hours or less, and preferably from 1 to 30 hours, more preferably from 3 to 10 hours. The product after completion of the reaction is appropriately distilled to obtain above-mentioned raw material olefin having 16 carbon atoms.

In an internal olefin sulfonic acid having 16 carbon atoms or a salt thereof as the component (A) obtained by sulfonation of the above-described raw material olefin having 16 carbon atoms, the content of the internal olefin sulfonic acid having a sulfonate group at the 1-position or more and the 4-position or less or a salt thereof in the component (A) is preferably 40 mass% or more, more preferably 50 mass% or more, and further more preferably 55 mass% or more and preferably 75 mass% or less, more preferably 70 mass% or less, and further more preferably 68 mass% or less. In addition, in the internal olefin sulfonic acid having 16 carbon atoms or a salt thereof as the component (A) obtained by sulfonation of the above-described raw material olefin having 16 carbon atoms, the content of the internal olefin sulfonic acid having a sulfonate group at the 1-position or more and the 4-position or less or a salt thereof in the component (A) is preferably 40 mass% or more and 75 mass% or less, more preferably from 50 to 70 mass%, and further more preferably from 55 to 68 mass%.

When the component (A) is sodium internal olefin sulfonate having 16 carbon atoms, the content of sodium internal olefin sulfonate having a sulfonate group at the 1-position or more and 4-position or less in the sodium internal olefin sulfonate having 16 carbon atoms is also same as above.

In the internal olefin sulfonic acid having 16 carbon atoms or a salt thereof as the component (A), the content of an internal olefin sulfonic acid having a sulfonate group at the 2-position or a salt thereof in the component (A) is preferably 10 mass% or more, more preferably 13 mass% or more, and further more preferably 17 mass% or more and preferably 35 mass% or less, more preferably 30 mass% or less, and further more preferably 25 mass% or less. In addition, in the internal olefin sulfonic acid having 16 carbon atoms or a salt thereof as the component (A), the content of the internal olefin sulfonic acid having a sulfonate group at the 2-position or a salt thereof in the component (A) is preferably 10 mass% or more and 35 mass% or less, more preferably from 13 to 30 mass%, and further more preferably from 17 to 25 mass%.

When the component (A) is sodium internal olefin sulfonate having 16 carbon atoms, in the sodium internal olefin sulfonate having 16 carbon atoms, the content of sodium internal olefin sulfonate having a sulfonate group at the 2-position is also same as above.

In the internal olefin sulfonic acid having 16 carbon atoms or a salt thereof as the component (A), the content of an internal olefin sulfonic acid having a sulfonate group at the 3-position or a salt thereof in the component (A) is preferably 5 mass% or more, more preferably 11 mass% or more, and further more preferably 15 mass% or more and preferably 30 mass% or less, more preferably 25 mass% or less, and further more preferably 20 mass% or less. In addition, in the internal olefin sulfonic acid having 16 carbon atoms or a salt thereof as the component (A), the content of the internal olefin sulfonic acid having a sulfonate group at the 3-position or a salt thereof in the component (A) is preferably 5 mass% or more and 30 mass% or less, more preferably from 11 to 25 mass%, and further more preferably from 15 to 20 mass%.

When the component (A) is sodium internal olefin sulfonate having 16 carbon atoms, the content of sodium internal olefin sulfonate having a sulfonate group at the 3-position in the sodium internal olefin sulfonate having 16 carbon atoms is also same as above.

In the internal olefin sulfonic acid having 16 carbon atoms or a salt thereof as the component (A), the content of an internal olefin sulfonic acid having a sulfonate group at the 4-position or a salt thereof in the component (A) is preferably 15 mass% or more, more preferably 18 mass% or more, and further more preferably 19 mass% or more and preferably 30 mass% or less, more preferably 25 mass% or less, and further more preferably 23 mass% or less. In addition, in the internal olefin sulfonic acid having 16 carbon atoms or a salt thereof as the component (A), the content of the internal olefin sulfonic acid having a sulfonate group at the 4-position or a salt thereof in the component (A) is preferably 15 mass% or more and 30 mass% or less, more preferably from 18 to 25 mass%, and further more preferably from 19 to 23 mass%.

When the component (A) is sodium internal olefin sulfonate having 16 carbon atoms, in the sodium internal olefin sulfonate having 16 carbon atoms, the content of sodium internal olefin sulfonate having a sulfonate group at the 4-position is also same as above.

In the internal olefin sulfonic acid having 16 carbon atoms or a salt thereof as the component (A), the content of an internal olefin sulfonic acid having a sulfonate group at the 1-position or a salt thereof in the component (A) is preferably less than 5.0 mass%, more preferably less than 3.0 mass%, and further more preferably less than 2.5 mass%. Alternatively, the internal olefin sulfonic acid having 16 carbon atoms or a salt thereof as the component (A) preferably does not contain the internal olefin sulfonic acid having a sulfonate group at the 1-position or a salt thereof.

When the component (A) is sodium internal olefin sulfonate having 16 carbon atoms, the content of sodium internal olefin sulfonate having a sulfonate group at the 1-position in the sodium internal olefin sulfonate having 16 carbon atoms is also same as above. Alternatively, it is preferable that the sodium internal olefin sulfonate having a sulfonate group at the 1-position is not contained.

In the internal olefin sulfonic acid having 16 carbon atoms or a salt thereof as the component (A), the mass ratio of the content of the hydroxy form (HAS) to the content of the olefin form (IOS), hydroxy form/olefin form, is preferably from 50/50 to 100/0, more preferably from 60/40 to 100/0, further more preferably from 70/30 to 100/0, further more preferably from 75/25 to 100/0, and more preferably from 75/25 to 95/5 from the viewpoint of productivity improvement and impurity reduction.

Such a mass ratio (hydroxy form/olefin form) is determined based on HPLC-MS peak areas that are obtained by separation of the hydroxy form and the olefin form from the component (A) by HPLC and application to MS.

When the component (A) is sodium internal olefin sulfonate having 16 carbon atoms, the mass ratio of the content of the hydroxy form (HAS) to the content of the olefin form (IOS), hydroxy form/olefin form, in the sodium internal olefin sulfonate having 16 carbon atoms is also same as above.

Since the internal olefin sulfonic acid having 16 carbon atoms or a salt thereof as the component (A) can be obtained by sulfonation of a raw material olefin, there is a possibility that the unreacted raw material olefin and the inorganic compound remain in the component (A). Lower contents of these components are preferable. The same applies also when the component (A) is sodium internal olefin sulfonate having 16 carbon atoms.

In the internal olefin sulfonic acid having 16 carbon atoms or a salt thereof as the component (A), the content of the unreacted raw material olefin in the component (A) is preferably less than 5.0 mass%, more preferably less than 3.0 mass%, further more preferably less than 1.5 mass%, and further more preferably less than 1.0 mass%.

When the component (A) is sodium internal olefin sulfonate having 16 carbon atoms, the content of the unreacted raw material olefin in the sodium internal olefin sulfonate having 16 carbon atoms is also same as above.

In the internal olefin sulfonic acid having 16 carbon atoms or a salt thereof as the component (A), the content of the inorganic compound in the component (A) is preferably less than 7.5 mass%, more preferably less than 5.0 mass%, further more preferably less than 3.0 mass%, further more preferably less than 2.0 mass%, and even more preferably less than 1.6 mass%.

When the component (A) is sodium internal olefin sulfonate having 16 carbon atoms, the content of the inorganic compound in the sodium internal olefin sulfonate having 16 carbon atoms is also same as above.

The content of the component (A) in the hair cleansing composition of the present invention is preferably 0.01 mass% or more, more preferably 0.05 mass% or more, further more preferably 0.1 mass% or more, further more preferably 0.7 mass% or more, and even more preferably 2 mass% or more from the viewpoint of effectively promoting the exhibition of excellent hair cleansing effect from the time of cleansing to the time of rinsing and after cleansing. Also, the content of the component (A) in the hair cleansing composition of the present invention is preferably 30 mass% or less, more preferably 15 mass% or less, further more preferably 12 mass% or less, further more preferably 10 mass% or less, and even more preferably 8 mass% or less from the viewpoint of more effectively expressing good combability in wet hair after cleansing and also expressing good low-temperature stability. In addition, the content of the component (A) in the hair cleansing composition of the present invention is preferably 0.01 mass% or more and 30 mass% or less, more preferably from 0.05 to 15 mass%, further more preferably from 0.1 to 12 mass%, further more preferably from 0.7 to 10 mass%, and even more preferably from 2 to 8 mass%.

The internal olefin sulfonic acid having 16 carbon atoms or a salt thereof as the component (A) can be obtained through sulfonation by reacting the raw material olefin having 16 carbon atoms with sulfur trioxide, specifically, by sulfonating the raw material olefin and then performing neutralization and subsequently hydrolysis.

More specifically, the amount of the sulfur trioxide that is used for sulfonation of the raw material olefin is preferably 0.8 mol or more, more preferably 0.9 mol or more, and further more preferably 0.95 mol or more based on 1 mol of the raw material olefin from the viewpoint of improving the yield of the component (A) and the viewpoint of improving the reactivity. In addition, the amount of the sulfur trioxide that is used for sulfonation of the raw material olefin is preferably 1.2 mol or less, more preferably 1.1 mol or less, and further more preferably 1.05 mol or less from the viewpoint of the economic efficiency and the viewpoint of suppressing unnecessary coloring of the component (A). In addition, the amount of the sulfur trioxide that is used for sulfonation of the raw material olefin is preferably from 0.8 to 1.2 mol, more preferably from 0.9 to 1.1 mol, and further more preferably from 0.95 to 1.05 mol based on 1 mol of the raw material olefin.

The reaction temperature when the raw material olefin is sulfonated is preferably 0°C or more from the viewpoint of preventing coagulation of the sulfur trioxide and the component (A) and is preferably 50°C or less from the viewpoint of suppressing unnecessary coloring of the component (A). The reaction temperature when the raw material olefin is sulfonated is preferably from 0°C to 50°C.

In the neutralization, an alkali compound, such as sodium hydroxide, potassium hydroxide, ammonia, and 2-aminoethanol, is used for the reaction. The amount of the alkali compound to be added is preferably 1.0 times mol or more, more preferably 1.03 times mol or more, per 1 mol of the sulfonate group from the viewpoint of suppressing the generation of impurities such as a raw material olefin and an inorganic salt and from the viewpoint of improving the reactivity. In addition, the amount of the alkali compound to be added is preferably 2.5 times mol or less, more preferably 2.0 times mol or less, and further more preferably 1.5 times mol or less per 1 mol of the sulfonate group from the viewpoint of the economic efficiency and the viewpoint of suppressing the generation of impurities such as a raw material olefin and an inorganic salt. The amount of the alkali compound to be added is preferably from 1.0 to 2.5 times mol, more preferably from 1.03 to 2.0 times mol, and further more preferably from 1.03 to 1.5 times mol per 1 mol of the sulfonate group.

In the neutralization, the temperature when the sulfonated raw material olefin and an alkali compound are mixed and the reaction temperature are preferably 40°C or less, more preferably 35°C or less, further more preferably 30°C or less, and further more preferably 25°C or less from the viewpoint of suppressing the generation of impurities, such as an internal olefin and an inorganic salt, by side reaction and are preferably 0°C or more, more preferably 10°C or more, further more preferably 15°C or more, and further more preferably 20°C or more from the viewpoint of improving the reactivity. In addition, the temperature when the sulfonated raw material olefin and an alkali compound are mixed and the reaction temperature are preferably from 0°C to 40°C, more preferably from 10°C to 35°C, further more preferably from 15°C to 30°C, and further more preferably from 20°C to 25°C.

The reaction temperature for hydrolysis that is performed after neutralization is preferably 120°C or more, more preferably 140°C or more, and further more preferably 160°C or more from the viewpoint of improving the reactivity in the presence of water. In addition, the reaction temperature for the hydrolysis is preferably 220°C or less and more preferably 180°C or less from the viewpoint of suppressing decomposition of the product. In addition, the reaction temperature for the hydrolysis is preferably from 120°C to 220°C, more preferably from 140°C to 180°C, and further more preferably from 160°C to 180°C.

The reaction time for the hydrolysis is preferably 30 minutes or more and more preferably 45 minutes or more from the viewpoint of completing the reaction. The reaction time for the hydrolysis is preferably 240 minutes or less, more preferably 180 minutes or less, further more preferably 120 minutes or less, and further more preferably 90 minutes or less from the viewpoint of improving the productivity. In addition, the reaction time for the hydrolysis is preferably from 30 to 240 minutes, more preferably from 45 to 180 minutes, further more preferably from 45 to 120 minutes, and further more preferably from 45 to 90 minutes. These reactions can be performed in series. After the completion of the reaction, purification can be performed by extraction, cleansing, and so on.

The same applies also when sodium internal olefin sulfonate having 16 carbon atoms is obtained as the component (A).

The hair cleansing composition of the present invention contains, as the component (B), one or more hydroxy group-containing compounds selected from the group consisting of (b1) a monosaccharide or a sugar alcohol and (b2) polyethylene glycol having a mass average molecular weight of 500 or less. By containing the component (B), it is possible to exhibit excellent hair cleansing effect and also secure good low-temperature stability in cooperation with the component (A) from the time of cleansing to the time of rinsing and after cleansing.

The monosaccharide or the sugar alcohol of the component (b1) is, from the viewpoint of the above effects, preferably a monosaccharide having from 4 to 6 carbon atoms, and is specifically one or more selected from the group consisting of monosaccharides such as glucose, fructose, mannose, galactose, and ribose. The sugar alcohol preferably has from 4 to 6 carbon atoms, and is specifically one or more sugar alcohols selected from the group consisting of mannitol, sorbitol, maltitol, lactitol, erythritol, and xylitol. Among these, from the viewpoint of exhibiting excellent hair cleansing effect from the time of cleansing to the time of rinsing and after cleansing, more specifically, one or more selected from the group consisting of glucose, mannitol, sorbitol, and erythritol are preferable, one or two selected from the group consisting of glucose and mannitol are more preferable, and those containing glucose are further more preferable.

Polyethylene glycol of the component (b2) has a mass average molecular weight of 500 or less, preferably a mass average molecular weight of 400 or less, more preferably a mass average molecular weight of 300 or less, and further more preferably a mass average molecular weight of 200 or less, and preferably has a mass average molecular weight of 100 or more. The "mass average molecular weight" means a value determined by the following expression (2):

Mass average molecular weight = {(56.1 × Number of functional groups)/Hydroxyl value} × 1 000          (2)

Among these components (b1) and (b2), the component (b2) is preferable from the viewpoint of effectively exhibiting excellent hair cleansing effect and from the viewpoint of securing good low-temperature stability from the time of cleansing to the time of rinsing and after cleansing.

The content of the component (B) in the hair cleansing composition of the present invention is preferably 0.05 mass% or more, more preferably 0.1 mass% or more, and further more preferably 0.5 mass% or more from the viewpoint of exhibiting excellent hair cleansing effect from the time of cleansing to the time of rinsing and after cleansing. Also, the content of the component (B) in the hair cleansing composition of the present invention is preferably 3.0 mass% or less, more preferably 2.5 mass% or less, and further more preferably 2.0 mass% or less from the viewpoint of effectively expressing good combability in wet hair after cleansing. In addition, the content of the component (B) in the hair cleansing composition of the present invention is preferably 0.05 mass% or more and 3.0 mass% or less, more preferably from 0.1 to 2.5 mass%, further more preferably from 0.1 to 2.0 mass%, and even more preferably from 0.5 to 2.0 mass%.

The mass ratio of the content of the component (B) to the content of the component (A), (B)/(A), is 0.005 or more and preferably 0.01 or more from the viewpoint of more effectively expressing good combability in wet hair after cleansing. Also, the mass ratio of the content of the component (B) to the content of the component (A), (B)/(A), is 0.45 or less, preferably 0.40 or less, and more preferably 0.35 or less from the viewpoint of effectively exhibiting excellent hair cleansing effect and securing good low-temperature stability from the time of cleansing to the time of rinsing and after cleansing. In addition, the mass ratio of the content of the component (B) to the content of the component (A), (B)/(A), is 0.005 or more and 0.45 or less, preferably from 0.005 to 0.40, more preferably from 0.01 to 0.40, and further more preferably from 0.01 to 0.35.

The hair cleansing composition of the present invention may contain a cationic polymer (C) from the viewpoint of, while securing good low-temperature stability, further ensuring the exhibition of excellent hair cleansing effect from the time of cleansing to the time of rinsing and after cleansing in cooperation with the above components. Here, the term "cationic polymer" means a polymer having a substituent that is positively ionized when dissolved in water.

Specifically, examples of the component (C) include one or more selected from the group consisting of cationated polygalactomannane, cationated hydroxyalkyl cellulose, diallyl quaternary ammonium salt polymers, copolymers containing methacrylamidopropyltrimethylammonium chloride, and crosslinked cationic polymers.

More specifically, examples of the cationated polygalactomannane include one or more selected from the group consisting of cationated guar gum, cationated tara gum, and cationated locust bean gum.

More specifically, examples of the cationated hydroxyalkyl cellulose include one or two selected from the group consisting of cationated hydroxyethyl cellulose, cationated hydroxypropyl cellulose, and so on.

More specifically, examples of the diallyl quaternary ammonium salt polymer include one or more selected from the group consisting of polydiallyldimethylammonium chloride, diallyldimethylammonium chloride/acrylic acid copolymers, diallyldimethylammonium chloride/acrylamide copolymers, and diallyldimethylammonium chloride/acrylic acid/acrylamide copolymers.

Examples of the copolymer containing methacrylamidopropyltrimethylammonium chloride include one or more selected from the group consisting of acrylic acid/methyl acrylate/methacrylamidopropyltrimethylammonium chloride copolymers and acrylic acid/acrylamide/methacrylamidopropyltrimethylammonium chloride copolymers.

More specifically, examples of the crosslinked cationic polymer include N,N-dimethylaminoethyl methacrylate diethyl sulfate/N,N-dimethylacrylamide/dimethacrylate polyethylene glycol copolymers.

In particular, one or more selected from the group consisting of cationated hydroxyalkyl cellulose and crosslinked cationic polymers are preferable, and cationated hydroxyethyl cellulose is more preferable.

The content of the component (C) in the hair cleansing composition of the present invention is preferably 0.01 mass% or more, more preferably 0.05 mass% or more, and further more preferably 0.1 mass% or more from the viewpoint of effectively promoting the exhibition of excellent hair cleansing effect from the time of cleansing to the time of rinsing and after cleansing. The content of the component (C) in the hair cleansing composition of the present invention is preferably 10 mass% or less, more preferably 3 mass% or less, further more preferably 1 mass% or less, and even more preferably 0.5 mass% or less from the viewpoint of securing good low-temperature stability and handleability. In addition, the content of the component (C) in the hair cleansing composition of the present invention is preferably from 0.01 to 10 mass%, more preferably from 0.05 to 3 mass%, further more preferably from 0.1 to 1 mass%, and further more preferably from 0.1 to 0.5 mass%.

The mass ratio of the content of the component (A) to the content of the component (C), (A)/(C), is preferably 1 or more, more preferably 5 or more, and further more preferably 10 or more from the viewpoint of exhibiting good low-temperature stability and handleability of the component (A). Also, the mass ratio of the content of the component (A) to the content of the component (C), (A)/(C), is preferably 1 000 or less, more preferably 500 or less, further more preferably 100 or less, further more preferably 50 or less, and even more preferably 30 or less from the viewpoint of effectively promoting the exhibition of excellent hair cleansing effect from the time of cleansing to the time of rinsing and after cleansing. In addition, the mass ratio of the content of the component (A) to the content of the component (C), (A)/(C), is preferably from 1 to 1 000, more preferably from 5 to 500, further more preferably from 10 to 100, further more preferably from 10 to 50, and even more preferably from 10 to 30.

The hair cleansing composition of the present invention may contain, as the component (D), an anionic surfactant other than the above component (A). By containing the component (D), it is possible to exhibit excellent hair cleansing effect while securing good low-temperature stability in cooperation with the component (A) from the time of cleansing to the time of rinsing and after cleansing.

Specifically, examples of the component (D) include one or more compounds selected from the group consisting of sulfonates, acylamino acid salts, sulfosuccinates, sulfuric ester salts, and carboxylates.

The sulfonate as the component (D) is a sulfonate other than the component (A), and more specifically, examples of the sulfonate include one or more selected from the group consisting of aminoethyl sulfonates such as N-acylmethyltaurate, alkyl benzene sulfonates, alkenyl benzene sulfonates, alkane sulfonates, and $\alpha$-olefin sulfonates having 14 carbon atoms. In particular, an alkyl group, an alkenyl group, or an acyl group preferably have from 6 to 22 carbon atoms, and an alkyl group, an alkenyl group, or acyl group preferably have from 8 to 18 carbon atoms.

More specifically, the number of carbon atoms in the acyl group of the acylamino acid salt is preferably from 6 to 22, and more preferably from 8 to 18. The amino acid moiety constituting the acylamino acid salt is preferably one or more selected from the group consisting of glutamic acid, aspartic acid, glycine, alanine, threonine, methylalanine, sarcosine, lysine, and arginine, and is more preferably one or more selected from the group consisting of glutamic acid, alanine, glycine, and arginine.

Examples of the sulfosuccinate include one or more selected from the group consisting of sulfosuccinic acid alkyl ester salts and polyoxyalkylene sulfosuccinic acid alkyl ester salts.

Examples of the sulfuric ester salt include one or more selected from the group consisting of alkyl sulfates, alkenyl sulfates, polyoxyalkylene alkyl ether sulfates, polyoxyalkylene alkenyl ether sulfates, polyoxyalkylene alkylphenyl ether sulfates and so on. In particular, the sulfuric ester salt preferably has an alkyl group or an alkenyl group having from 6 to 22 carbon atoms, and more preferably an alkyl group or an alkenyl group having from 8 to 18 carbon atoms.

Examples of the carboxylate include one or more selected from the group consisting of fatty acid salts, polyoxyalkylene alkyl ether acetates, and so on. In particular, the carboxylate preferably has an alkyl group, an alkenyl group, or an aliphatic acid group having from 6 to 22 carbon atoms, and more preferably has an alkyl group, an alkenyl group, or an aliphatic acid group having from 8 to 18 carbon atoms.

Among these components (D), from the viewpoint of exhibiting excellent hair cleansing effect from the time of cleansing to the time of rinsing and after cleansing, while securing good low-temperature stability, one or more selected from the group consisting of aminoethyl sulfonate, acylamino acid salts, sulfosuccinates, polyoxyalkylene alkyl ether sulfates, and polyoxyalkylene alkyl ether acetates are preferable, and one or more selected from the group consisting of aminoethyl sulfonate, acylamino acid salts, sulfosuccinates, and polyoxyalkylene alkyl ether sulfates are more preferable, and aminoethyl sulfonate is further more preferable.

The content of the component (D) in the hair cleansing composition of the present invention is preferably 0.01 mass% or more, more preferably 0.05 mass% or more, further more preferably 0.1 mass% or more, further more preferably 0.8 mass% or more, and even more preferably 1.5 mass% or more from the viewpoint of effectively promoting the exhibition of excellent hair cleansing effect from the time of cleansing to the time of rinsing and after cleansing. Also, the content of the component (D) in the hair cleansing composition of the present invention is preferably 30 mass% or less, more preferably 15 mass% or less, further more preferably 12 mass% or less, further more preferably 10 mass% or less, further more preferably 9.3 mass% or less, and even more preferably 8.0 mass% or less from the viewpoint of more effectively expressing good combability in wet hair after cleansing and from the viewpoint of securing good low-temperature stability. In addition, the content of the component (D) in the hair cleansing composition of the present invention is preferably 0.01 mass% or more and 30 mass% or less, more preferably from 0.05 to 15 mass%, further more preferably from 0.1 to 12 mass%, further more preferably from 0.8 to 10 mass%, further more preferably from 0.8 to 9.3 mass%, further more preferably from 0.8 to 8.0 mass%, and even more preferably from 1.5 to 8.0 mass%.

When the component (D) contains an internal olefin sulfonic acid or a salt thereof other than those having 16 carbon atoms, the mass ratio of the content of the component (A) to the total amount of the internal olefin sulfonic acids or salts thereof in the hair cleansing composition of the present invention, (A)/(total amount of internal olefin sulfonic acids or salts thereof), is preferably 0.2 or more, more preferably 0.4 or more, further more preferably 0.6 or more, further more preferably 0.8 or more, further more preferably 0.85 or more, further more preferably 0.9 or more, and even more preferably 0.95 or more from the viewpoint of providing good foaming at the time of cleansing.

The mass ratio of the content of the component (A) to the content of the component (D), (A)/(D), is preferably 0.01 or more, more preferably 0.05 or more, further more preferably 0.1 or more, and even more preferably 0.3 or more from the viewpoint of more effectively expressing good combability in wet hair after cleansing and from the viewpoint of securing good low-temperature stability. Also, the mass ratio of the content of the component (A) to the content of the component (D), (A)/(D), is preferably 100 or less, more preferably 25 or less, further more preferably 15 or less, further more preferably 10 or less, further more preferably 8 or less, and even more preferably 5 or less from the viewpoint of exhibiting excellent hair cleansing effect. In addition, the mass ratio of the content of the component (A) to the content of the component (D), (A)/(D), is preferably 0.01 or more and 100 or less, more preferably from 0.05 to 25, further more preferably from 0.1 to 15, further more preferably from 0.1 to 10, further more preferably from 0.1 to 8, further more preferably from 0.3 to 8, and even more preferably from 0.3 to 5.

The hair cleansing composition of the present invention can contain an amphoteric surfactant (E) from the viewpoint of further ensuring the exhibition of excellent hair cleansing effect coupled with the component (A).

Specifically, examples of the component (E) include one or more selected from the group consisting of carbobetaines and sulfobetaines. More specifically, the examples include one or more selected from the group consisting of carbobetaines and sulfobetaines each including an alkyl group, alkenyl group, or acyl group having from 6 to 22 carbon atoms, preferably from 8 to 18 carbon atoms.

In particular, one or more selected from the group consisting of lauramidopropyl betaine, cocamidopropyl betaine, lauramidopropyl hydroxysulfobetaine, and lauryl sulfobetaine are preferable, and one or two selected from the group consisting of lauramidopropyl betaine and lauramidopropyl hydroxysulfobetaine are more preferable.

The content of the component (E) in the hair cleansing composition of the present invention is preferably 0.01 mass% or more, more preferably 0.05 mass% or more, further more preferably 0.1 mass% or more, and even more preferably 0.3 mass% or more from the viewpoint of further enhancing the exhibition of excellent hair cleansing effect. The content of the component (E) in the hair cleansing composition of the present invention is preferably 30 mass% or less, more preferably 15 mass% or less, further more preferably 12 mass% or less, further more preferably 8 mass% or less, and even more preferably 5 mass% or less from the viewpoint of further certainly ensuring good combability in wet hair after cleansing and from the viewpoint of securing good low-temperature stability. In addition, the content of the component (E) in the hair cleansing composition of the present invention is preferably from 0.01 to 30 mass%, more preferably from 0.05 to 15 mass%, further more preferably from 0.1 to 12 mass%, further more preferably from 0.1 to 8 mass%, further more preferably from 0.1 to 5 mass%, and even more preferably from 0.3 to 5 mass%.

The mass ratio of the content of the component (A) to the content of the component (E), (A)/(E), is preferably 0.01 or more, more preferably 0.05 or more, and further more preferably 0.1 or more from the viewpoint of more effectively expressing good combability in wet hair after cleansing. Also, the mass ratio of the content of the component (A) to the content of the component (E), (A)/(E), is preferably 50 or less, more preferably 25 or less, further more preferably 9 or less, and even more preferably 5 or less from the viewpoint of effectively enhancing excellent hair cleansing effect and securing good low-temperature stability. In addition, the mass ratio of the content of the component (A) to the content of the component (E), (A)/(E), is preferably from 0.01 to 50, more preferably from 0.05 to 25, further more preferably from 0.1 to 9, and even more preferably from 0.1 to 5.

The hair cleansing composition of the present invention can contain a nonionic surfactant (F) from the viewpoint of further ensuring the exhibition of excellent hair cleansing effect coupled with the above-mentioned components.

Specifically, examples of the component (F) include one or more selected from the group consisting of polyoxyalkylene alkyl ether, fatty acid alkanolamide, alkyl glycoside, and alkyl glyceryl ether.

More specifically, examples of the polyoxyalkylene alkyl ether include those of which the alkyl group has from 6 to 22 carbon atoms, preferably from 8 to 18 carbon atoms. In particular, polyoxyethylene alkyl ether is preferable, and those of which the oxyethylene group has an average number of moles added of from 3 to 50, more preferably from 4 to 16, are further preferable.

Examples of the fatty acid alkanolamide include mono- or di- alkanolamide of which the fatty acid has from 6 to 22 carbon atoms, preferably from 8 to 18 carbon atoms.

Examples of the alkyl glycoside include those of which the alkyl group has from 6 to 22 carbon atoms, preferably from 8 to 18 carbon atoms.

Examples of the alkyl glyceryl ether include those of which the alkyl group has from 6 to 22 carbon atoms, preferably from 8 to 18 carbon atoms.

In particular, fatty acid alkanolamide is more preferable.

The content of the component (F) in the hair cleansing composition of the present invention is preferably 0.01 mass% or more, more preferably 0.05 mass% or more, further more preferably 0.1 mass% or more, and even more preferably 0.5 mass% or more from the viewpoint of further enhancing the exhibition of excellent hair cleansing effect and from the viewpoint of securing good low-temperature stability in cooperation with the component (B). The content of the component (F) in the hair cleansing composition of the present invention is preferably 15 mass% or less, more preferably 10 mass% or less, further more preferably 8 mass% or less, and even more preferably 6 mass% or less from the viewpoint of further securing good combability in wet hair after cleansing in cooperation with the component (B). In addition, the content of the component (F) in the hair cleansing composition of the present invention is preferably from 0.01 to 15 mass%, more preferably from 0.05 to 10 mass%, further more preferably from 0.1 to 8 mass%, and further more preferably from 0.5 to 6 mass%.

The mass ratio of the content of the component (A) to the content of the component (F), (A)/(F), is preferably 0.01 or more, more preferably 0.05 or more, further more preferably 0.1 or more, and even more preferably 0.5 or more from the viewpoint of effectively suppressing unnecessary precipitation of the component (A) to further secure good combability in wet hair after cleansing and from the viewpoint of securing good low-temperature stability. Also, the mass ratio of the content of the component (A) to the content of the component (F), (A)/(F), is preferably 50 or less, more preferably 25 or less, further more preferably 9 or less, and even more preferably 6 or less from the viewpoint of exhibiting excellent hair cleansing effect. In addition, the mass ratio of the content of the component (A) to the content of the component (F), (A)/(F), is preferably from 0.01 to 50, more preferably from 0.05 to 25, further more preferably from 0.1 to 9, further more preferably from 0.5 to 9, and even more preferably from 0.5 to 6.

The hair cleansing composition of the present invention can contain, in addition to the above-mentioned components, water that can be a medium when the component (A) is obtained by sulfonation of a raw material olefin, a viscosity reducer, polyhydric alcohols, a preservative, and a reducing agent, and also other components that are usually used as cosmetic raw materials, within range that do not impair the effects of the present invention. Examples of such components include a texture improver, a thickener, perfume, a UV absorber, a visible light absorber, a chelating agent, an antioxidant, a colorant, a preservative, a pH adjuster, a viscosity adjuster, a pearlescent agent, and a wetting agent.

The pH of the hair cleansing composition of the present invention at 25°C is, as the pH of a 5% water dispersion, preferably 3.0 or more, more preferably 3.2 or more, and further more preferably 4.3 or more from the viewpoint of foaming at the time of cleansing and from the viewpoint of securing good low-temperature stability. The pH of the hair cleansing composition of the present invention at 25°C is, as the pH of a 5% water dispersion, preferably 7.0 or less, more preferably 6.5 or less, and further more preferably 6.0 or less from the viewpoint of suppressing entanglement of hair and so on. In addition, the pH of the hair cleansing composition of the present invention at 25°C is, as the pH of a 5% water dispersion, preferably from 3.0 to 7.0, more preferably from 3.2 to 6.5, and further more preferably from 4.3 to 6.0.

The hair cleansing composition of the present invention usually contains an aqueous medium. Examples of the aqueous medium include water; lower alcohol, such as ethanol and isopropyl alcohol; and low molecular weight diol and triol having 6 or less carbon atoms, such as 1,3-butylene glycol, glycerin, ethylene glycol, and propylene glycol, and water is preferable. Although the content of the aqueous medium can be appropriately selected depending on the dosage form of the hair cleansing composition, the content in the hair cleansing composition is usually from 5 to 99 mass% and preferably from 30 to 98 mass%.

To apply the hair cleansing composition of the present invention to hair, specifically a method in which the hair is moistened with water in advance, cleansed by applying the hair cleansing composition of the present invention to the hair, and then rinsed with water may be used, for example. Such a method is also highly useful as a method for enhancing combability in wet hair after cleansing.

Thus, the hair cleansing composition of the present invention when applied to hair exhibits performance of expressing good foaming at the time of cleansing and imparting softness to the hair at the time of rinsing and, also, unlikely causes entanglement of wet hair after cleansing, thus enabling good combability to be felt.

Accordingly, the hair cleansing composition of the present invention is highly useful especially as a hair cleansing composition for improving combability in wet hair after cleansing.

Regarding the above-described embodiments, the present invention further discloses the following hair cleansing compositions:

[1] A hair cleansing composition comprising the following components (A) and (B):

(A) an internal olefin sulfonic acid having 16 carbon atoms or a salt thereof obtained by sulfonation of a raw material olefin having 16 carbon atoms and having an average double bond position of 3.9-position or more and 4.4-position or less; and
(B) one or more hydroxy group-containing compounds selected from the group consisting of (b1) a monosaccharide or a sugar alcohol and (b2) polyethylene glycol having a mass average molecular weight of 500 or less,
wherein a mass ratio of a content of the component (B) to a content of the component (A), (B)/(A), is 0.005 or more and 0.45 or less;

[2] The hair cleansing composition according to the above [1], wherein in the internal olefin sulfonic acid having 16 carbon atoms or a salt thereof as the component (A), the content of the internal olefin sulfonic acid having a sulfonate group at the 1-position or more and the 4-position or less or a salt thereof in the component (A) is preferably 40 mass% or more, more preferably 50 mass% or more, and further more preferably 55 mass% or more and preferably 75 mass% or less, more preferably 70 mass% or less, and further more preferably 68 mass% or less;

[3] The hair cleansing composition according to the above [1] or [2], wherein in the internal olefin sulfonic acid having 16 carbon atoms or a salt thereof as the component (A), the content of the internal olefin sulfonic acid having a sulfonate group at the 2-position or a salt thereof in the component (A) is preferably 10 mass% or more, more preferably 13 mass% or more, and further more preferably 17 mass% or more and preferably 35 mass% or less, more preferably 30 mass% or less, and further more preferably 25 mass% or less;

[4] The hair cleansing composition according to any one of the above [1] to [3], wherein in the internal olefin sulfonic acid having 16 carbon atoms or a salt thereof as the component (A), the content of the internal olefin sulfonic acid having a sulfonate group at the 3-position or a salt thereof in the component (A) is preferably 5 mass% or more, more preferably 11 mass% or more, and further more preferably 15 mass% or more and preferably 30 mass% or less, more preferably 25 mass% or less, and further more preferably 20 mass% or less;

[5] The hair cleansing composition according to any one of the above [1] to [4], wherein in the internal olefin sulfonic acid having 16 carbon atoms or a salt thereof as the component (A), the content of the internal olefin sulfonic acid having a sulfonate group at the 4-position in the component (A) is preferably 15 mass% or more, more preferably 18 mass% or more, and further more preferably 19 mass% or more and preferably 30 mass% or less, more preferably 25 mass% or less, and further more preferably 23 mass% or less;

[6] The hair cleansing composition according to any one of the above [1] to [5], wherein the average double bond position of the raw material olefin having 16 carbon atoms which forms the component (A) is 3.9-position or more and preferably 4.0-position or more, and 4.4-position or less, preferably 4.3-position or less, and more preferably 4.2-position or less;

[7] The hair cleansing composition according to any one of the above [1] to [6], wherein in the raw material olefin having 16 carbon atoms which forms the component (A), the content of the raw material olefin having a double bond position at the 2-position in the raw material olefin having 16 carbon atoms is preferably 10 mass% or more, more preferably 15 mass% or more, and further more preferably 20 mass% or more and preferably 35 mass% or less, more preferably 32 mass% or less, and further more preferably 24 mass% or less;

[8] The hair cleansing composition according to any one of the above [1] to [7], wherein in the internal olefin sulfonic acid having 16 carbon atoms or a salt thereof as the component (A), the mass ratio of the content of the hydroxy form (HAS) to the content of the olefin form (IOS), hydroxy form/olefin form, is preferably from 50/50 to 100/0, more preferably from 60/40 to 100/0, further more preferably from 70/30 to 100/0, further more preferably from 75/25 to 100/0, and more preferably from 75/25 to 95/5.

[9] The hair cleansing composition according to any one of the above [1] to [8], wherein the content of the component (A) in the hair cleansing composition of the present invention is preferably 0.01 mass% or more, more preferably 0.05 mass% or more, further more preferably 0.1 mass% or more, further more preferably 0.7 mass% or more, and even more preferably 2 mass% or more and preferably 30 mass% or less, more preferably 15 mass% or less, further more preferably 12 mass% or less, further more preferably 10 mass% or less, and even more preferably 8 mass% or less;

[10] The hair cleansing composition according to any one of the above [1] to [9], wherein the monosaccharide or the sugar alcohol of the component (b1) is preferably a monosaccharide or a sugar alcohol having from 4 to 6 carbon atoms, more preferably one or more selected from the group consisting of glucose, fructose, mannose, galactose, ribose, mannitol, sorbitol, erythritol, xylose, and pentaerythritol, further more preferably one or more selected from the group consisting of glucose, mannitol, sorbitol, and erythritol, even more preferably one or two selected from the group consisting of glucose and mannitol, and even more preferably one containing glucose;

[11] The hair cleansing composition according to any one of the above [1] to [10], wherein the polyethylene glycol of the component (b2) has a mass average molecular weight of preferably 400 or less, more preferably 300 or less, and further more preferably 200 or less and preferably 100 or more;

[12] The hair cleansing composition according to any one of the above [1] to [11], wherein the content of the component (B) in the hair cleansing composition of the present invention is preferably 0.05% by mass or more, more preferably 0.1% by mass or more, and further more preferably 0.5% by mass or more and preferably 3.0% by mass or less, more preferably 2.5% by mass or less, and further more preferably 2.0% by mass or less;

[13] The hair cleansing composition according to any one of the above [1] to [12], wherein the mass ratio of the content of the component (B) to the content of the component (A), (B)/(A), is preferably 0.01 or more, and preferably 0.40 or less and more preferably 0.35 or less.

[14] The hair cleansing composition according to any one of the above [1] to [13], further comprising a cationic polymer (C), wherein the component (C) is preferably one or more selected from the group consisting of cationated polygalactomannane, cationated hydroxyalkyl cellulose, diallyl quaternary ammonium salt polymers, methacrylamide propyl trimethyl ammonium chloride-containing copolymers, and crosslinked cationic polymers, more preferably one or more selected from the group consisting of cationated hydroxyalkyl cellulose and crosslinked cationic polymers, and further more preferably cationated hydroxyethyl cellulose;

[15] The hair cleansing composition according to the above [14], wherein the content of the component (C) in the hair cleansing composition of the present invention is preferably 0.01% by mass or more, more preferably 0.05% by mass or more, and further more preferably 0.1% by mass or more and preferably 10% by mass or less, more preferably 3% by mass or less, further more preferably 1% by mass or less, and even more preferably 0.5% by mass or less, and the mass ratio of the content of the component (A) to the content of the component (C), (A)/(C), is preferably 1 or more, more preferably 5 or more, and further more preferably 10 or more and preferably 1 000 or less, more preferably 500 or less, further more preferably 100 or less, further more preferably 50 or less, and even more preferably 30 or less;

[16] The hair cleansing composition according to any one of the above [1] to [15], further comprising an anionic surfactant (D) other than the component (A) and the component (B), wherein the component (D) is preferably one or more selected from the group consisting of sulfonates, acylamino acid salts, sulfosuccinates, sulfuric ester salts, and carboxylates.

[17] The hair cleansing composition according to the above [16], wherein the content of the component (D) in the hair cleansing composition of the present invention is preferably 0.01 mass% or more, more preferably 0.05 mass% or more, further more preferably 0.1 mass% or more, further more preferably 0.8 mass% or more, and even more preferably 1.5 mass% or more and preferably 30 mass% or less, more preferably 15 mass% or less, further more preferably 12 mass% or less, further more preferably 10 mass% or less, further more preferably 9.3 mass% or less, and even more preferably 8.0 mass% or less;

[18] The hair cleansing composition according to the above [16] or [17], wherein when an internal olefin sulfonic acid or a salt thereof other than those having 16 carbon atoms is included as the component (D), the mass ratio of the content of the component (A) to the total amount of the internal olefin sulfonic acids or salts thereof in the hair cleansing composition of the present invention, (A)/(total amount of internal olefin sulfonic acids or salts thereof), is preferably 0.2 or more, more preferably 0.4 or more, further more preferably 0.6 or more, further more preferably 0.8 or more, further more preferably 0.85 or more, further more preferably 0.9 or more, and even more preferably 0.95 or more;

[19] The hair cleansing composition according to any one of the above [16] to [18], wherein the mass ratio of the content of the component (A) to the content of the component (D), (A)/(D), is preferably 0.01 or more, more preferably 0.05 or more, further more preferably 0.1 or more, and even more preferably 0.3 or more and preferably 100 or less, more preferably 25 or less, further more preferably 15 or less, further more preferably 10 or less, further more preferably 8 or less, and even more preferably 5 or less;

[20] The hair cleansing composition according to any one of the above [1] to [19], further comprising an amphoteric surfactant (E), wherein the component (E) is preferably one or more selected from the group consisting of lauramidopropyl betaine, cocamidopropyl betaine, lauramidopropyl hydroxysulfobetaine, and lauryl sulfobetaine, and more preferably one or two selected from the group consisting of lauramidopropyl betaine and lauramidopropyl hydroxysulfobetaine;

[21] The hair cleansing composition according to the above [20], wherein the mass ratio of the content of the component (A) to the content of the component (E), (A)/(E), is preferably 0.01 or more, more preferably 0.05 or more, and further more preferably 0.1 or more and preferably 50 or less, more preferably 25 or less, further more preferably 9 or less, and even more preferably 5 or less;

[22] The hair cleansing composition according to any one of the above [1] to [21], further comprising a nonionic surfactant (F), wherein the component (F) is one or more selected from the group consisting of polyoxyalkylene alkyl ether, fatty acid alkanolamide, alkyl glycoside, and alkyl glyceryl ether;

[23] The hair cleansing composition according to the above [22], wherein the mass ratio of the content of the component (A) to the content of the component (F), (A)/(F), is preferably 0.01 or more, more preferably 0.05 or more, further more preferably 0.1 or more, and even more preferably 0.5 or more and preferably 50 or less, more preferably 25 or less, further more preferably 9 or less, and even more preferably 6 or less;

[24] The hair cleansing composition according to any one of the above [1] to [23], wherein the pH at 25°C is, as the pH of a 5% water dispersion, preferably 3.0 or more, more preferably 3.2 or more, and further more preferably 4.3 or more and preferably 7.0 or less, more preferably 6.5 or less, and further more preferably 6.0 or less;

[25] The hair cleansing composition according to any one of the above [1] to [24], which is used to improve combability in wet hair after cleansing;

[26] A cleansing method for improving combability in wet hair after cleansing, comprising the steps in which the hair is moistened with water in advance, washed by applying the hair cleansing composition according to any one of the above [1] to [24] to the hair, and then rinsed with water;

[27] Use of the hair cleansing composition according to any one of the above [1] to [24] to improve combability in wet hair after cleansing;

[28] A method of improving combability in wet hair after cleansing, in which the hair is moistened with water in advance, washed by applying the hair cleansing composition according to any one of the above [1] to [24] to the hair, and then rinsed with water;

[29] A method of improving softness of hair at the time of rinsing, in which the hair is moistened with water in advance, washed by applying the hair cleansing composition according to any one of the above [1] to [24] to the hair, and then rinsed with water.

[30] Use of the hair cleansing composition according to any one of the above [1] to [24] to improve softness of hair at the time of rinsing.

Examples

The present invention will now be specifically described based on examples. Unless otherwise indicated in the table, the content of each component indicates mass%.

[Measurement methods for various physical properties]

(i) Method for measuring double bond position of raw material olefin

The double bond position of a raw material olefin was measured by gas chromatography (hereinafter, abbreviated as GC). Specifically, a dithiolated derivative of a raw material olefin was obtained by reaction with dimethyl disulfide, and then each component was separated by GC. As a result, the double bond positions of the raw material olefin were determined from the respective peak areas.

The apparatuses and analysis conditions used for measurement are as follows: GC apparatus (trade name: HP6890, manufactured by Hewlett-Packard Company); column (trade name: Ultra-Alloy-1HT capillary column 30 m × 250 $\mu$m × 0.15 $\mu$m, manufactured by Frontier Laboratories Ltd.); detector (hydrogen flame ionization detector (FID)); injection temperature: 300°C; detector temperature: 350°C; and flow rate of He: 4.6 mL/min.

(ii) Method for measuring content according to sulfonate group bonding position of sodium internal olefin sulfonate

Regarding sodium internal olefin sulfonate with an attached sulfonate group, each sodium internal olefin sulfonate content according to the sulfonate group bonding position was measured by high performance liquid chromatography/mass spectrometer (HPLC-MS). Specifically, hydroxy forms with a sulfonate group attached were separated by high performance liquid chromatography (HPLC) and were each applied to mass spectrometer (MS) for identification. As a result, each content was determined from the HPLC-MS peak areas.

The apparatuses and conditions used for measurement were as follows: HPLC apparatus "LD20ASXR" (manufactured by Shimadzu Corporation); column "ODS Hypersil (registered trademark)" (4.6 × 250 mm, particle size: 3 μm, manufactured by Thermo Fisher Scientific); sample preparation (1 000 times dilution with methanol); eluent A (10 mM ammonium acetate added water); eluent B (10 mM ammonium acetate added methacrylonitrile/water = 95/5 (v/v) solution); gradient (0 minutes (A/B = 60/40) → 15.1 to 20 minutes (30/70) → 20.1 to 30 minutes (60/40)); MS apparatus "LCMS-2020" (manufactured by Shimadzu Corporation); ESI detector (anion detection m/z: 321.10 (A) component having 16 or 18 carbon atoms); column temperature (40°C); flow rate (0.5 mL/min); and injection volume (5 μL).

(iii) Method for measuring mass ratio of hydroxy form/olefin form

The mass ratio of hydroxy form/olefin form of sodium internal olefin sulfonate was measured by HPLC-MS. Specifically, the hydroxy form and the olefin form were separated by HPLC and were respectively applied to MS for identification. As a result, the rate of each of them was determined from the HPLC-MS peak areas.

The apparatuses and conditions used for the measurement were as follows: HPLC apparatus (trade name: Agilent Technology 1100, manufactured by Agilent Technologies, Inc.); column (trade name: L-column ODS 4.6 × 150 mm, manufactured by Chemicals Evaluation and Research Institute, Japan); sample preparation (1 000 times dilution with methanol); eluent A (10 mM ammonium acetate added water); eluent B (10 mM ammonium acetate added methanol); gradient (0 minutes (A/B = 30%/70%) → 10 minutes (30%/70%) → 55 minutes (0%/100%) → 65 minutes (0%/100%) → 66 minutes (30%/70%) → 75 minutes (30%/70%)); MS apparatus (trade name: Agilent Technology 1100 MS SL (G1946D), manufactured by Agilent Technologies, Inc.); and MS detection (anion detection m/z 60-1600, UV 240 nm).

(iv) Method for measuring content of raw material olefin

The content of unreacted raw material olefin in the sodium internal olefin sulfonate was measured by GC. Specifically, ethanol and petroleum ether were added to a sodium internal olefin sulfonate aqueous solution, and extraction was then performed to obtain olefin in the petroleum ether phase. As a result, the raw material olefin was quantitatively measured from the GC peak area.

The apparatuses and analysis conditions used for the measurement were as follows: GC apparatus (trade name: Agilent Technology 6850, manufactured by Agilent Technologies, Inc.); column (trade name: Ultra-Alloy-1HT capillary column 15 m × 250 μm × 0.15 μm, manufactured by Frontier Laboratories Ltd.); detector (hydrogen flame ionization detector (FID)); injection temperature: 300°C; detector temperature: 350°C; and He flow rate: 3.8 mL/min.

(v) Method for measuring content of inorganic compound

The contents of inorganic compounds were measured by potentiometric titration and neutralization titration. Specifically, the content of $Na_2SO_4$ was quantitatively measured by determining the sulfate radical ($SO_4^{2-}$) by potentiometric titration. The content of NaOH was quantitatively measured by neutralization titration with dilute hydrochloric acid.

[Manufacturing Example a1: manufacturing of raw material olefin a1 having 16 carbon atoms]

1-Hexadecanol (product name: KALCOL 6098, manufactured by Kao Corporation) 7 000 g (28.9 mol) and γ-alumina (manufactured by STREM Chemicals, Inc.) 350 g (5 mass% with respect to the raw material alcohol) as a solid acid catalyst were placed in a flask equipped with a stirrer, and reaction was performed by stirring at 280°C for 8 hours while circulating nitrogen (7 000 mL/min) in the system. The alcohol conversion rate after the completion of the reaction was 100%. The obtained crude alkene internal olefin was transferred to a distillation flask and was distilled at 136°C to 160°C/4.0 mmHg to obtain a raw material olefin a1 having 16 carbon atoms with an olefin purity of 100%. The double bond distribution of the obtained raw material olefin a1 was C1-position: 1.8 mass%, C2-position: 21.8 mass%, C3-position: 18.7 mass%, C4-position: 18.6 mass%, C5-position: 14.3 mass%, C6-position: 11.4 mass%, and the total of C7- and C8-positions: 13.6 mass%, and the average double bond position was 4.17.

[Manufacturing Example a2: manufacturing of raw material olefin a2 having 16 carbon atoms]

A raw material olefin a2 having 16 carbon atoms was obtained with an olefin purity of 100% as in Manufacturing Example a1 except that the reaction time was changed to 7.5 hours. The double bond distribution of the obtained raw material olefin a2 was C1-position: 2.4 mass%, C2-position: 23.2 mass%, C3-position: 18.7 mass%, C4-position: 18.2 mass%, C5-position: 13.9 mass%, C6-position: 11.2 mass%, and the total of C7- and the 8-positions: 12.4 mass%, and the average double bond position was 4.08.

[Manufacturing Example a3: manufacturing of raw material olefin a3 having 16 carbon atoms]

A raw material olefin a3 having 16 carbon atoms was obtained with an olefin purity of 100% as in Manufacturing Example a1 except that the reaction time was changed to 8.5 hours. The double bond distribution of the obtained raw material olefin a3 was C1-position: 2.3 mass%, C2-position: 20.7 mass%, C3-position: 16.8 mass%, C4-position: 17.5 mass%, C5-position: 14.7 mass%, C6-position: 12.9 mass%, and the total of C7- and 8-positions: 15.2 mass%, and the average double bond position was 4.28.

[Manufacturing Example a4: manufacturing of raw material olefin a4 having 16 carbon atoms]

A raw material olefin a4 having 16 carbon atoms was obtained with an olefin purity of 100% as in Manufacturing Example a1 except that the reaction time was changed to 6.5 hours. The double bond distribution of the obtained raw material olefin a4 was C1-position: 2.3 mass%, C2-position: 29.7 mass%, C3-position: 22.7 mass%, C4-position: 17.3 mass%, C5-position: 11.1 mass%, C6-position: 8.0 mass%, and the total of C7- and 8-positions: 9.0 mass%, and the average double bond position was 3.70.

[Manufacturing Example a5: manufacturing of raw material olefin a5 having 16 carbon atoms]

A raw material olefin a5 having 16 carbon atoms was obtained with an olefin purity of 100% as in Manufacturing Example a1 except that the reaction time was changed to 9.5 hours. The double bond distribution of the obtained raw material olefin a5 was C1-position: 0.9 mass%, C2-position: 19.2 mass%, C3-position: 16.1 mass%, C4-position: 15.7 mass%, C5-position: 16.6 mass%, C6-position: 813.2 mass%, and the total of C7- and 8-positions: 18.4 mass%, and the average double bond position was 4.50.

[Manufacturing Example a6: manufacturing of raw material olefin a6 having 18 carbon atoms]

1-Octadecanol (product name: KALCOL 8098, manufactured by Kao Corporation) 7 000 g (25.9 mol) and γ-alumina (manufactured by STREM Chemicals, Inc.) 700 g (10 mass% with respect to the raw material alcohol) as a solid acid catalyst were placed in a flask equipped with a stirrer, and reaction was performed by stirring at 280°C for 11 hours while circulating nitrogen (7 000 mL/min) in the system. The alcohol conversion rate after the completion of the reaction was 100%. The obtained crude alkene internal olefin was transferred to a distillation flask and was distilled at 148°C to 158°C/0.5 mmHg to obtain a raw material olefin a6 having 18 carbon atoms with an olefin purity of 100%. The double bond distribution of the obtained raw material olefin a6 was C1-position: 1.8 mass%, C2-position: 26.4 mass%, C3-position: 21.1 mass%, C4-position: 17.5 mass%, C5-position: 11.7 mass%, C6-position: 8.3 mass%, C7-position: 5.9 mass%, and the total of C8- and 9-positions: 7.4 mass%, and the average double bond position was 4.00.

Each of the physical property values of the obtained raw material olefins a1 to a6 are shown in Table 1.

[Table 1]

| Raw material olefin | | a1 | a2 | a3 | a4 | a5 | a6 |
|---|---|---|---|---|---|---|---|
| Number of carbon atoms | | 16 | 16 | 16 | 16 | 16 | 18 |
| Double bond distribution in raw material olefin (mass%) | 1-position | 1.8 | 2.4 | 2.3 | 2.3 | 0.9 | 1.8 |
| | 2-position | 21.8 | 23.2 | 20.7 | 29.7 | 19.2 | 26.4 |
| | 3-position | 18.7 | 18.7 | 16.8 | 22.7 | 16.1 | 21.1 |
| | 4-position | 18.6 | 18.2 | 17.5 | 17.3 | 15.7 | 17.5 |
| | 5-position | 14.3 | 13.9 | 14.7 | 11.1 | 16.6 | 11.7 |
| | 6-position | 11.4 | 11.2 | 12.9 | 8.0 | 13.2 | 8.3 |
| | 7-position | 6.8 | 6.2 | 7.6 | 4.5 | 9.2 | 5.9 |
| | 8-position | 6.8 | 6.2 | 7.6 | 4.5 | 9.2 | 3.7 |
| | 9-position | | | | | | 3.7 |
| | Total | 100.0 | 100.0 | 100.0 | 100.0 | 100.0 | 100.0 |
| Average double bond position (DBP) | | 4.17 | 4.08 | 4.28 | 3.70 | 4.50 | 4.00 |

[Manufacturing Example 1: manufacturing of sodium internal olefin sulfonate A1 having 16 carbon atoms]

The raw material olefin a1 obtained in Manufacturing Example a1 was put in a thin film sulfonation reactor having an outer jacket, and sulfonation reaction was performed using a sulfur trioxide gas under the condition of allowing cooling water of 10°C to pass through the reactor outer jacket. The molar ratio of $SO_3$/internal olefin during the sulfonation reaction was set to 1.01. The obtained sulfonated product was mixed with an alkali aqueous solution prepared with sodium hydroxide (alkali agent) in an amount of 1.04 times mol per mol of the theoretical acid value, and neutralization was performed by a continuous method at 30°C for 1 hour. The obtained neutralized product was heated in an autoclave at 170°C for 1 hour for hydrolysis to obtain sodium internal olefin sulfonate A1 having 16 carbon atoms. The content of the raw material olefin contained in the obtained sodium internal olefin sulfonate A1 having 16 carbon atoms was 0.4 mass%, and the content of the inorganic compound was 0.39 mass%.

[Manufacturing Example 2: manufacturing of sodium internal olefin sulfonate A2 having 16 carbon atoms]

Sodium internal olefin sulfonate A2 having 16 carbon atoms was obtained as in Manufacturing Example 1 except that the raw material olefin a2 obtained in Manufacturing Example a2 was used as the raw material olefin. The content of the raw material olefin contained in the obtained sodium internal olefin sulfonate A2 having 16 carbon atoms was 0.7 mass%, and the content of the inorganic compound was 0.49 mass%.

[Manufacturing Example 3: manufacturing of sodium internal olefin sulfonate A3 having 16 carbon atoms]

Sodium internal olefin sulfonate A3 having 16 carbon atoms was obtained as in Manufacturing Example 1 except that the raw material olefin a3 obtained in Manufacturing Example a3 was used as the raw material olefin. The content of the raw material olefin contained in the obtained sodium internal olefin sulfonate A3 having 16 carbon atoms was 0.5 mass%, and the content of the inorganic compound was 0.54 mass%.

[Manufacturing Example 4: manufacturing of sodium internal olefin sulfonate A4 having 16 carbon atoms]

Sodium internal olefin sulfonate A4 having 16 carbon atoms was obtained as in Manufacturing Example 1 except that the raw material olefin a4 obtained in Manufacturing Example a4 was used as the raw material olefin. The content of the raw material olefin contained in the obtained sodium internal olefin sulfonate A4 having 16 carbon atoms was 0.4 mass%, and the content of the inorganic compound was 0.41 mass%.

[Manufacturing Example 5: manufacturing of sodium internal olefin sulfonate A5 having 16 carbon atoms]

Sodium internal olefin sulfonate A5 having 16 carbon atoms was obtained as in Manufacturing Example 1 except that the raw material olefin a5 obtained in Manufacturing Example a5 was used as the raw material olefin. The content of the raw material olefin contained in the obtained sodium internal olefin sulfonate A5 having 16 carbon atoms was 0.3 mass%, and the content of the inorganic compound was 0.43 mass%.

[Manufacturing Example 6: manufacturing of sodium internal olefin sulfonate A6 having 18 carbon atoms]

Sodium internal olefin sulfonate A6 having 18 carbon atoms was obtained as in Manufacturing Example 1 except that the raw material olefin a6 obtained in Manufacturing Example a6 was used as the raw material olefin. The content of the raw material olefin contained in the obtained sodium internal olefin sulfonate A6 having 18 carbon atoms was 0.5 mass%, and the content of the inorganic compound was 0.45 mass%.

Each of the physical property values of the obtained sodium internal olefin sulfonates A1 to A6 are shown in Table 2.

[Table 2]

| Sodium internal olefin sulfonate | A1 | A2 | A3 | A4 | A5 | A6 |
|---|---|---|---|---|---|---|
| Raw material olefin | a1 | a2 | a3 | a4 | a5 | a6 |

(continued)

| Sodium internal olefin sulfonate | | A1 | A2 | A3 | A4 | A5 | A6 |
|---|---|---|---|---|---|---|---|
| Distribution of sulfonate group (mass%) | 1-position | 2.0 | 1.2 | 1.6 | 1.6 | 0.8 | 1.4 |
| | 2-position | 24.8 | 18.7 | 17.5 | 18.4 | 13.9 | 20.7 |
| | 3-position | 19.1 | 16.1 | 15.7 | 15.2 | 12.1 | 17.4 |
| | 4-position | 22.0 | 19.9 | 20.3 | 19.3 | 18.1 | 21.0 |
| | 5- to 9-positions | 32.1 | 44.2 | 45.0 | 45.5 | 55.0 | 39.6 |
| | Total | 100 | 100 | 100 | 100 | 100 | 100 |
| Hydroxy form | | 83.9 | 84.2 | 83.8 | 84.0 | 84.5 | 83.8 |
| Olefin form | | 16.1 | 15.8 | 16.2 | 16.0 | 15.5 | 16.2 |

[Examples 1 to 13 and Comparative Examples 1 to 6]

Hair cleansing compositions having the compositions shown in Tables 3 to 4 were prepared by a usual method using the obtained sodium internal olefin sulfonate A1. Specifically, a component (A), a component (B), an appropriate amount of water, a component (C) as needed, and other components were placed in a beaker and were heated to 60 to 80°C, mixed, and cooled to room temperature. Subsequently, water was replenished, and the pH was adjusted to 6.0 with a pH adjuster (succinic acid or disodium succinate aqueous solution) to obtain each hair cleansing composition.

Each evaluation was performed using the obtained hair cleansing compositions according to the following methods.

The results are shown in Tables 3 to 4.

<<Evaluation of "good foaming" at the time of cleansing and "softness of hair" at the time of rinsing>>

The components shown below were placed in a beaker and were heated to 80°C and then mixed, and after confirmation of uniform dissolution, cooled to obtain plain shampoo.

(Composition of plain shampoo)

| (Component) | (mass%) |
|---|---|
| Na polyoxyethylene lauryl ether sulfate (42.0% as EMAL E-27C (manufactured by Kao Corporation, active component: 27 mass%)) | 11.3 |
| Coconut fatty acid N-methylethanolamide (AMINON C-11S (manufactured by Kao Corporation)) | 3.0 |
| Citric acid | 0.2 |
| Methylparaben | 0.3 |
| Purified water | balance |
| Total | 100.0 |

A bundle of untreated hair having a mass of 20 g and a length of 20 cm of a Japanese was cleansed with the above plain shampoo to obtain a tress for evaluation. The obtained tress for evaluation was sufficiently wetted with warm water at 35°C to 40°C, and 1 g of any of the hair cleansing compositions was then applied to the tress, followed by cleansing for 1 minute. Each of the items of "good foaming" at the time of cleansing, and "softness of hair" at the time of rinsing, were evaluated by one special panelist according to the following evaluation criteria.

a: Very good,
b: Good
c: Bad.

<<Evaluation of "good combability" in wet hair after cleansing>>

The above tress for evaluation was provided and sufficiently wetted with warm water at 35 to 40°C, then 1 g of any of the hair cleaning compositions was applied, the tress was lightly held between the palms of both hands and washed for 1 minute by moving the hands so as to rub the hands against each other. Then, the tress was rinsed with warm water for 30 seconds to obtain a tress for combability evaluation.

The resulting tress was placed in a combing force measuring apparatus ("KOT-0303" manufactured by Utsunomiya Seiki), and a brush was moved downward from the root to the tip of the tress. The maximum load (gf) applied to the brush when the brush traveled from the root to the tip was measured 10 times, and the average value thereof was evaluated as the maximum value of combing force (gf).

A smaller value of the maximum load on the brush means that the entanglement of wet hair after cleansing is more effectively prevented, spontaneously providing aligned hair flow, and thus brushing and subsequent drying are more easily performed.

<<Evaluation of low-temperature stability>>

The obtained hair cleansing compositions were put in respective screw bottles and were stored in a thermostat chamber of -5°C. Subsequently, whether precipitate was formed over time was visually checked and evaluated according to the following criteria:

a: Formation of precipitate was not observed even after the lapse of 6 hours;
b: Formation of precipitate was observed during the lapse of 5 hours or more and less than 6 hours; and
c: Formation of precipitate was observed before the lapse of less than 5 hours.

[Table 3]

| | | Example | Example | Example | Example | Example | Example | Example | Example | Comparative Example | Comparative Example | Comparative Example |
|---|---|---|---|---|---|---|---|---|---|---|---|---|
| | | 1 | 2 | 3 | 4 | 5 | 6 | 7 | 8 | 1 | 2 | 3 |
| (A) | Sodium C16 internal olefin sulfonate A1 | 7.00 | 7.00 | 7.00 | 7.00 | 7.00 | 7.00 | 7.00 | 7.00 | 7.00 | | 7.00 |
| (D) | Sodium methyl cocoyl taurate*1 | 3.00 | 3.00 | 3.00 | 3.00 | 3.00 | 3.00 | 3.00 | 3.00 | 3.00 | 3.00 | 3.00 |
| (E) | Lauramidopropyl hydroxysultaine*2 | 2.70 | 2.70 | 2.70 | 2.70 | 2.70 | 2.70 | 2.70 | 2.70 | 2.70 | 2.70 | 2.70 |
| | Lauramidopropyl betaine*3 | 0.30 | 0.30 | 0.30 | 0.30 | 0.30 | 0.30 | 0.30 | 0.30 | 0.30 | 0.30 | 0.30 |
| (F) | Polyoxyethylene (6) lauryl ether*4 | 1.00 | 1.00 | 1.00 | 1.00 | 1.00 | 1.00 | 1.00 | 1.00 | 1.00 | 1.00 | 1.00 |
| | N-Cocoyl-methyl ethanol amide*5 | 1.20 | 1.20 | 1.20 | 1.20 | 1.20 | 1.20 | 1.20 | 1.20 | 1.20 | 1.20 | 1.20 |
| | Lauryl glucoside*6 | 1.70 | 1.70 | 1.70 | 1.70 | 1.70 | 1.70 | 1.70 | 1.70 | 1.70 | 1.70 | 1.70 |
| | 2-Ethylhexyl glyceryl ether*7 | 0.11 | 0.11 | 0.11 | 0.11 | 0.11 | 0.11 | 0.11 | 0.11 | 0.11 | 0.11 | 0.11 |
| (C) | Cationated hydroxyethyl cellulose X*8 | 0.10 | 0.10 | 0.10 | 0.10 | 0.10 | 0.10 | 0.10 | 0.10 | 0.10 | 0.10 | 0.10 |
| | Cationated hydroxyethyl cellulose Y*9 | 0.35 | 0.35 | 0.35 | 0.35 | 0.35 | 0.35 | 0.35 | 0.35 | 0.35 | 0.35 | 0.35 |
| (B) | Polyethylene glycol (M.W.=200)*10 | 0.05 | 0.10 | 0.50 | 1.00 | 1.50 | 2.00 | 2.50 | 3.00 | 3.50 | 1.00 | |
| | Preservative | 0.10 | 0.10 | 0.10 | 0.10 | 0.10 | 0.10 | 0.10 | 0.10 | 0.10 | 0.10 | 0.10 |
| | Solvent | 0.75 | 0.75 | 0.75 | 0.75 | 0.75 | 0.75 | 0.75 | 0.75 | 0.75 | 0.75 | 0.75 |
| | pH adjuster | 0.20 | 0.20 | 0.20 | 0.20 | 0.20 | 0.20 | 0.20 | 0.20 | 0.20 | 0.20 | 0.20 |
| | Purified water | Balance | Balance | Balance | Balance | Balance | Balance | Balance | Balance | Balance | Balance | Balance |

(continued)

| | | Example | Example | Example | Example | Example | Example | Example | Example | Comparative Example | Comparative Example | Comparative Example |
|---|---|---|---|---|---|---|---|---|---|---|---|---|
| | | 1 | 2 | 3 | 4 | 5 | 6 | 7 | 8 | 1 | 2 | 3 |
| | Total | 100.00 | 100.00 | 100.00 | 100.00 | 100.00 | 100.00 | 100.00 | 100.00 | 100.00 | 100.00 | 100.00 |
| | (B)/(A) | 0.007 | 0.014 | 0.07 | 0.14 | 0.21 | 0.29 | 0.36 | 0.43 | 0.50 | - | 0.00 |
| Foaming | | a | a | a | a | a | a | b | b | b | b | a |
| Softness of hair at the time of rinsing | | a | a | a | a | a | a | a | a | b | c | b |
| Maximum value of combing force (gf) | | 213 | 122 | 101 | 148 | 107 | 190 | 229 | 279 | 361 | 354 | 432 |
| Low-temperature stability | | a | a | a | a | a | a | a | a | a | a | a |

*1: DIAPON K-SF, manufactured by NOF Corporation
*2: SOFTAZOLINE LSB-R, manufactured by Kawaken Fine Chemicals Co., Ltd.
*3: SOFTAZOLINE LPB-R, manufactured by Kawaken Fine Chemicals Co., Ltd.
*4: EMULGEN 108, manufactured by Kao Corporation
*5: AMINON C-11S, manufactured by Kao Corporation
*6: AG-124, manufactured by Kao Corporation
*7: PENETOL GE-EH, manufactured by Kao Corporation
*8: UCARE Polymer LR-400 (Polyquaternium -10), manufactured by The Dow Chemical Company
*9: SoftCAT Polymer SL-30 (Polyquaternium -67), manufactured by The Dow Chemical Company
*10: PEG#200, manufactured by NOF Corporation

[Table 4]

| | | Example | Example | Example | Example | Example | Example | Comp. Ex. | Comp. Ex. | Comp. Ex. |
|---|---|---|---|---|---|---|---|---|---|---|
| | | 9 | 10 | 4 | 11 | 12 | 13 | 4 | 5 | 6 |
| (A) | Sodium C16 internal olefin sulfonate A1 (DBP4.17, C16) | 7.00 | 7.00 | 7.00 | 7.00 | | | | | 7.00 |
| | Sodium C16 internal olefin sulfonate A2 (DBP4.08, C16) | | | | | 7.00 | | | | |
| | Sodium C16 internal olefin sulfonate A3 (DBP4.28, C16) | | | | | | 7.00 | | | |
| | Sodium C16 internal olefin sulfonate A4 (DBP3.70, C16) | | | | | | | 7.00 | | |
| | Sodium C16 internal olefin sulfonate A5 (DBP4.50, C16) | | | | | | | | 7.00 | |
| | Sodium C16 internal olefin sulfonate A6 (DBP4.00, C18) | | | | | | | | | 0.50 |
| (D) | Sodium methyl cocoyl taurate[*1] | 3.00 | 3.00 | 3.00 | 3.00 | 3.00 | 3.00 | 3.00 | 3.00 | 3.00 |
| (E) | Lauramidopropyl hydroxysultaine[*2] | 2.70 | 2.70 | 2.70 | 2.70 | 2.70 | 2.70 | 2.70 | 2.70 | 2.70 |
| | Lauramidopropyl betaine[*3] | 0.30 | 0.30 | 0.30 | 0.30 | 0.30 | 0.30 | 0.30 | 0.30 | 0.30 |
| (F) | Polyoxyethylene (6) lauryl ether[*4] | 1.00 | 1.00 | 1.00 | 1.00 | 1.00 | 1.00 | 1.00 | 1.00 | 1.00 |
| | N-Cocoyl-methyl ethanol amide[*5] | 1.20 | 1.20 | 1.20 | 1.20 | 1.20 | 1.20 | 1.20 | 1.20 | 1.20 |
| | Lauryl glucoside[*6] | 1.70 | 1.70 | 1.70 | 1.70 | 1.70 | 1.70 | 1.70 | 1.70 | 1.70 |
| | 2-Ethylhexyl glyceryl ether[*7] | 0.11 | 0.11 | 0.11 | 0.11 | 0.11 | 0.11 | 0.11 | 0.11 | 0.11 |
| (C) | Cationated hydroxyethyl cellulose X[*8] | 0.10 | 0.10 | 0.10 | 0.10 | 0.10 | 0.10 | 0.10 | 0.10 | 0.10 |
| | Cationated hydroxyethyl cellulose Y[*9] | 0.35 | 0.35 | 0.35 | 0.35 | 0.35 | 0.35 | 0.35 | 0.35 | 0.35 |
| (B) | Glucose[*11] | 1.00 | | | | | | | | |
| | Mannitol[*12] | | 1.00 | | | | | | | |
| | Polyethylene glycol (M.W.=200)[*10] | | | 1.00 | | 1.00 | 1.00 | 1.00 | 1.00 | 1.00 |
| | Polyethylene glycol (M.W.=400)[*13] | | | | 1.00 | | | | | |
| | Preservative | 0.10 | 0.10 | 0.10 | 0.10 | 0.10 | 0.10 | 0.10 | 0.10 | 0.10 |
| | Solvent | 0.75 | 0.75 | 0.75 | 0.75 | 0.75 | 0.75 | 0.75 | 0.75 | 0.75 |

(continued)

|  |  | Example 9 | Example 10 | Example 4 | Example 11 | Example 12 | Example 13 | Comp. Ex. 4 | Comp. Ex. 5 | Comp. Ex. 6 |
|---|---|---|---|---|---|---|---|---|---|---|
|  | pH adjuster | 0.20 | 0.20 | 0.20 | 0.20 | 0.20 | 0.20 | 0.20 | 0.20 | 0.20 |
|  | Purified water | Balance | Balance | Balance | Balance | Balance | Balance | Balance | Balance | Balance |
|  | Total | 100.00 | 100.00 | 100.00 | 100.00 | 100.00 | 100.00 | 100.00 | 100.00 | 100.00 |
|  | (B)/(A) | 0.14 | 0.14 | 0.14 | 0.14 | 0.14 | 0.14 | 0.14 | 0.14 | 0.13 |
| Foaming | | a | a | a | a | a | a | a | a | a |
| Softness of hair at the time of rinsing | | a | a | a | a | a | a | a | b | a |
| Maximum value of combing force (gf) | | 170 | 249 | 148 | 240 | 209 | 161 | 205 | 316 | 141 |
| Low-temperature stability | | a | a | a | a | a | a | c | a | c |

*1-10: Same as Table 3
*11: manufactured by FUJIFILM Wako Pure Chemical Corporation
*12: Marine Crystal, manufactured by Mitsubishi Corporation Life Sciences Limited
*13: manufactured by FUJIFILM Wako Pure Chemical Corporation

**Claims**

1. A hair cleansing composition comprising the following components (A) and (B):

   (A) an internal olefin sulfonic acid having 16 carbon atoms or a salt thereof obtained by sulfonation of a raw material olefin having 16 carbon atoms and having an average double bond position of 3.9-position or more and 4.4-position or less; and
   (B) one or more hydroxy group-containing compounds selected from the group consisting of (b1) a monosaccharide or a sugar alcohol and (b2) polyethylene glycol having a mass average molecular weight of 500 or less,

   wherein a mass ratio of a content of the component (B) to a content of the component (A), (B)/(A), is 0.005 or more and 0.45 or less.

2. The hair cleansing composition according to claim 1, wherein the component (b1) is one or more selected from the group consisting of glucose, fructose, mannose, galactose, ribose, mannitol, sorbitol, erythritol, xylose, and pentaerythritol.

3. The hair cleansing composition according to claim 1 or 2, wherein a content of an internal olefin sulfonic acid or a salt thereof including a sulfonate group at the 1-position or more and the 4-position or less in the component (A) is 40 mass% or more and 75 mass% or less.

4. The hair cleansing composition according to any one of claims 1 to 3, wherein a content of an internal olefin sulfonic acid or a salt thereof including a sulfonate group at the 2-position in the component (A) is 10 mass% or more and 35 mass% or less.

5. The hair cleansing composition according to any one of claims 1 to 4, wherein a content of an internal olefin sulfonic acid or a salt thereof including a sulfonate group at the 3-position in the component (A) is 5 mass% or more and 30 mass% or less.

6. The hair cleansing composition according to any one of claims 1 to 5, wherein a content of the component (B) is 0.05 mass% or more and 1.5 mass% or less.

7. The hair cleansing composition according to any one of claims 1 to 6, wherein a mass ratio of a content of a hydroxy form of the internal olefin sulfonic acid or a salt thereof to a content of an olefin form of the internal olefin sulfonic acid or a salt thereof, (hydroxy form/olefin form), in the component (A) is from 50/50 to 100/0.

8. The hair cleansing composition according to any one of claims 1 to 7, wherein a content of the component (A) is 0.01 mass% or more and 30 mass% or less.

9. The hair cleansing composition according to any one of claims 1 to 8, further comprising a cationic polymer (C).

10. The hair cleansing composition according to any one of claims 1 to 9, for improving combability on wet hair after cleansing.

## INTERNATIONAL SEARCH REPORT

| International application No. |
| --- |
| **PCT/JP2023/004498** |

**A. CLASSIFICATION OF SUBJECT MATTER**

*A61K 8/46*(2006.01)i; *A61K 8/34*(2006.01)i; *A61K 8/60*(2006.01)i; *A61K 8/86*(2006.01)i; *A61Q 5/02*(2006.01)i
FI: A61K8/46; A61K8/34; A61K8/60; A61K8/86; A61Q5/02

According to International Patent Classification (IPC) or to both national classification and IPC

**B. FIELDS SEARCHED**

Minimum documentation searched (classification system followed by classification symbols)

A61K8/46; A61K8/34; A61K8/60; A61K8/86; A61Q5/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); Japio-GPG/FX

**C. DOCUMENTS CONSIDERED TO BE RELEVANT**

| Category* | Citation of document, with indication, where appropriate, of the relevant passages | Relevant to claim No. |
| --- | --- | --- |
| X | JP 2015-027977 A (KAO CORP) 12 February 2015 (2015-02-12)<br>claims, paragraphs [0009], [0012], [0059], [0068], [0096], [0127], [0169], [0181], [0188], [0207]-[0209] | 1-10 |
| A | JP 2015-27976 A (KAO CORP) 12 February 2015 (2015-02-12)<br>entire text | 1-10 |
| A | JP 2018-203628 A (ICHIMARU PHARCOS INC) 27 December 2018 (2018-12-27)<br>entire text | 1-10 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **10 April 2023** | **25 April 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**
Information on patent family members

International application No.

**PCT/JP2023/004498**

| Patent document cited in search report | | | Publication date (day/month/year) | Patent family member(s) | | | Publication date (day/month/year) |
|---|---|---|---|---|---|---|---|
| JP | 2015-027977 | A | 12 February 2015 | US<br>claims, paragraphs [0014],<br>[0022], [0059], [0066], [0087],<br>[0121], [0126], [0134], [0160]-<br>[0161], table 1<br>WO | 2014/0079660<br><br><br><br><br>2014/046300 | A1<br><br><br><br><br>A2 | |
| JP | 2015-27976 | A | 12 February 2015 | US<br>entire text<br>WO | 2014/0076344<br><br>2014/046299 | A1<br><br>A2 | |
| JP | 2018-203628 | A | 27 December 2018 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2020109145 A

- JP 2017214571 A

**Non-patent literature cited in the description**

- *J. Am. Oil Chem. Soc.*, 1992, vol. 69, 39